# EUROPÄISCHE PATENTSCHRIFT

(11) **EP 0 709 105 B1**
(45) Veröffentlichungstag und Bekanntmachung des Hinweises auf die Patenterteilung: **05.12.2001**
(21) Anmeldenummer: 95109071.1
(22) Anmeldetag: 13.06.1995
(51) Int. Cl.: A61M 5/28, A61M 5/34, A61M 5/31

(54) **Vorfüllbare partikelarme, sterile Einmalspritze für die Injektion von Präparaten und Verfahren zu ihrer Herstellung**
Pre-filled sterile disposable syringe with low quantity of particles for injection of compositions and method for its manufacture
Seringue pré-remplie, stérile à usage unique et faible quantité de particles pour l'injection des compositions et procédé pour sa fabrication

(30) Priorität: 27.10.1994 DE 4438360
(43) Veröffentlichungstag der Anmeldung: 01.05.1996
(73) Patentinhaber: Schott Glas, 55122 Mainz (DE)
(72) Erfinder: Reinhard, Michael, Dr., D-55270 Ober-Olm (DE); Spallek, Michael, Dr., D-55218 Ingelheim (DE)
(74) Vertreter: Fuchs Mehler Weiss & Fritzsche

(56) Entgegenhaltungen:
- EP-A- 0 556 034
- WO-A-94/15581
- WO-A-95/12482
- DE-A- 3 916 101
- US-A- 3 330 004
- DATABASE WPI Section Ch, Week 8730 Derwent Publications Ltd., London, GB; Class A96, AN 87-210130 & JP-A-62 137 065 (ISONO) , 19.Juni 1987

## Beschreibung

Die Erfindung betrifft eine vorfüllbare partikelarme, sterile Einmalspritze für die Injektion von Präparaten mit einem Füllvolumen kleiner als 5 ml, bestehend aus einem Spritzenkörper, der einen innen mit einer Gleitschicht versehenen Spritzenzylinder mit einem offenen Ende zur Aufnahme eines Spritzenkolbens, der mittels einer Kolbenstange im Spritzenzylinder bewegbar ist, eine Griffleiste und einen Spritzenkopf aufweist, und einer Injektionsnadel, die im Spritzenkopf unlösbar befestigt ist, derart, daß die Nadel in die Schutzkappe einsticht und damit verschlossen ist, während gleichzeitig die Innenseite des Kappenhemdes dichtend am Spritzenkopf anliegt, wodurch auch die äußere Fläche der Nadel abgedichtet ist. Die Erfindung betrifft ferner ein Verfahren zur Herstellung einer solchen Spritze.

Vorgefüllte sterile Einmalspritzen für medizinische Zwecke vereinen zwei verschiedene Funktionen. Zum einen sind sie dazu geeignet, die eingefüllten hochwirksamen Präparate über mehrere Jahre sicher zu lagern; zum anderen sind sie aber auch das Instrument, mit dem die Präparate direkt dem Patienten verabreicht werden. Während bei Fläschchen und Ampullen das Präparat zuerst dem Behältnis mittels einer Leerspritze entnommen werden muß und erst dann verabreicht werden kann, entfällt dieser Umfüllvorgang, d.h. das Aufziehen der Spritze, bei der vorgefüllten Einmalspritze. Dadurch werden der Arbeitsaufwand, die Verwechslungsgefahr und die Kontaminationsgefahr erheblich reduziert. Außerdem stellt die direkte Verabreichung aus dem Behältnis unter dem Gesichtspunkt "Total Cost of Drug Delivery" die mit Abstand konstengünstigste Möglichkeit dar.

Bisher ist nur eine Ausführung der eingangs bezeichneten vorfüllbaren Einmalspritze bekannt. Es ist die SCF - Staked Needle Spritze der Firma Becton Dickinson, wobei SCF für Sterile Clean Fill steht. Der Spritzenkörper, d.h. der Spritzenzylinder, die Griffleiste und der Spritzenkopf bestehen einteilig aus Glas, weshalb die Spritze auch als vorfüllbare Glas-Einmalpritze bezeichnet wird. Überhaupt besteht bei allen bekannten Einmalspritzen für die Injektion mit einem Füllvolumen < 5 ml zumindest der Spritzenzylinder aus Glas.

Bei der SCF - Staked Needle Spritze ist die Nadel in eine entsprechende Ausnehmung im Spritzenkopf eingeklebt. Es sind auch Glas-Einmalspritzen bekannt geworden (DE 29 39 180 C2; DE 39 16 101), bei denen die Injektionsnadel nicht unmittelbar in den Spritzenkopf integriert, sondern in ein Ansatzstück eingeklebt ist, das mechanisch in den Spritzenkopf verbindbar ist. Dadurch wird die Anzahl der Teile erhöht, und es entsteht eine zusätzliche Fügestelle, die dicht sein muß.

Ein gravierender Nachteil der Glas-Einmalspritze ist die Bruchgefahr und die damit verbundene Verletzungs- und Infektionsgefahr. Ferner sind manche Gläser nicht für eine Gammasterilisierung geeignet, da eine bleibende Verfärbung des Glases eintritt. Die Gammasterilisierung ist aber ein sehr einfaches, kostengünstiges und unbedenkliches Sterilisationsverfahren.

Ein weiterer Nachteil der Glas-Einmalspritze sind die aufwendigen Prozesse, die notwendig sind, um die Glasteile herzustellen und in einen partikelarmen und sterilen Zustand zu überführen. Der Herstellungsprozeß für die Glasteile ist langsam und nur in Ansätzen Rechner-steuerbar bzw. -kontrollierbar. Entsprechend groß ist die Prozeßunsicherheit. Hinzu kommt, daß die Glasteile immer mit Werkzeugen und Werkzeugschmierung in Berührung kommen, weshalb ein Waschvorgang zwingend notwendig ist. Die entsprechenden Waschanlagen erfordern hohe Investitionen und ihr Betrieb ist teuer.

Es sind auch vorfüllbare Einmalspritzen aus Kunststoff bekannt, deren Füllvolumen mindestens 50 ml beträgt. Bei der unter dem Warenzeichen OPTIRAY bekannt gewordenen Injektor-Einmalspritze der Fa. Mallinckrodt ist keine Injektionsnadel vorgesehen, sondern es wird ein Infusionsschlauch auf dem Spritzenkopf mechanisch befestigt. Der Kunststoff besteht aus (transluzentem) PP.

Bei der Einmalspritze gemäß der US-A-4,861,335 ist der aus Kunststoff bestehende Spritzenzylinder auf der dem Spritzenkolben abgewandten Seite mit einer Kunststoffkappe versehen, in der mittels einer unter dem Warenzeichen LUER-LOCK bekanntgewordenen Befestigungsart die Injektionsnadel gehaltert ist. Die Nadel ist damit nicht im Spritzenkopf integriert.

Bei Spritzen mit Füllvolumina deutlich unter 50 ml, insbesondere im Bereich von kleiner als 5 ml, ist die Oberfläche der Spritze im Vergleich zum abgefüllten Volumen so groß, daß die bekannten Kunststoffspritzen vorgefüllt nicht langzeitlagerfähig wären. Die Verluste durch das Herausdiffundieren von Bestandteilen des Präparates, insbesondere des Lösungsmittels Wasser, wären so groß, daß sich die Zusammensetzung unzulässig ändert. Ferner sind die bekannten Kunststoffspritzen aus einem transluzenten Kunststoff aufgebaut, der nur eine beschränkte visuelle Kontrolle des Spritzeninhaltes zuläßt, eine Maßnahme, die gerade bei kleinvolumigen vorgefüllten Einmalspritzen von Bedeutung ist. Daher sind im Bereich von Füllvolumen unterhalb 5 ml nur vorgefüllte Glas-Einmalspritzen bekannt.

Der Erfindung liegt die Aufgabe zugrunde, eine vorfüllbare Einmalspritze zu schaffen, bei der gegenüber einer Ausführung in Glas eine deutlich geringere Bruch- und Verletzungsgefahr besteht, die eine hohe Barriere gegen Wasserdampf-Diffusion aufweist, die eine optische Inspektion des abgefüllten Präparates zuläßt und bei der der Prozeß zur Herstellung der vormontierten partikelarm und steril verpackten Einheit, bestehend aus Spritzenkörper mit integrierter Nadel und aufgesetzter Schutzkappe, besonders einfach, gut beherrschbar und kostengünstig ist.

Die Lösung dieser Aufgabe gelingt mit einer Einmalspritze gemäß Anspruch 1.

Durch die erfindungsgemäßen Maßnahmen gelingt es, eine kleinvolumige, vorfüllbare sterile Einmalspritze der eingangs genannten Gattung zu schaffen, die trotz der Verwendung von Kunststoff als Werkstoff langzeitlagerfähig ist, eine visuelle Kontrolle des Spritzeninhaltes auf Ausfällungen, Verunreinigungen etc. erlaubt, d.h. alle Vorteile der Glas-Einmalspritze besitzt, ohne jedoch die durch die Verwendung des Werkstoffes Glas bedingten Handhabungs- und Fertigungsnachteile in Kauf nehmen zu müssen.

Die Handhabung beim Applizieren des Spritzeninhaltes ist wesentlich sicherer als bei Glas-Einmalspritzen.

Hinsichtlich des Verfahrens zum Herstellen der erfindungsgemäßen vorfüllbaren sterilen Einmalspritze gelingt die Lösung der vorgenannten Aufgabe mit einem Verfahren gemäß Anspruch 12.

Dieser Prozeß zur Herstellung der vormontierten, partikelarm und steril verpackten Einheit, bestehend aus Spritzenkörper mit integrierter Nadel und aufgesetzter Schutzkappe, ist besonders einfach, gut beherrschbar und kostengünstig. Er ist auch leichter als im bekannten Fall automatisierbar. Die vormontierte Einheit wird dann dem Pharmazeuten angeliefert, der den Spritzenzylinder befüllt und vollmontiert (Einsetzen des Kolbens), ohne daß aufwendige zusätzliche Verfahrensschritte notwendig wären.

Eine Ausgestaltung der erfindungsgemäßen Einmalspritze besteht darin, daß der Bereich des Spritzenkopfes, in dem die Injektionsnadel gehaltert ist, als Umspritzung der Nadel ausgeführt ist. Diese Umspritzung der Nadel hat den Vorteil gegenüber den bekannten Glas-Fertigspritzen mit eingeklebter Nadel, daß kein Klebstoff mit dem Präparat in Wechselwirkung treten kann, was besonders bei hochempfindlichen Präparaten von Bedeutung ist.

Der reibungsbedingte Kaftschluß zwischen Nadel und Umspritzung reicht im Regelfall aus, um eine sichere Halterung der Injektionsnadel zu gewährleisten. Vorteilhaft ist es jedoch, wenn die Injektionsnadel zur Herstellung einer formschlüssigen Sicherung der Nadelposition im umspritzten Bereich eine Profilierung oder Verformung aufweist, die z.B. S-förmig ausgestaltet sein kann. Hier stehen dem Fachmann zahlreiche Möglichkeiten zur Erzielung einer Abweichung von der schlank-zylindrischen Form, bis hin zur Anbringung einer Verdickung an der Nadel, zur Verfügung.

Alternativ zur Umspritzung ist bei einer anderen Ausführungsform der Erfindung die Nadel in eine Ausnehmung am Spritzenkopf eingeklebt. Diese Einmalspritze mit eingeklebter Nadel kann bei weniger empfindlichen Präparaten eingesetzt werden und hat den Vorteil geringerer Herstellkosten. Das Spritzgießen ohne Einlegteile vereinfacht den Werkzeugaufbau und macht kürzere Zykluszeiten möglich. Auch wird das nachträgliche Einkleben der Nadel mit hohen Taktfrequenzen sicher beherrscht.

Eine weitere Ausgestaltung der Erfindung besteht darin, daß in dem Bereich des Spritzenkopfes, in dem die Injektionsnadel gehalten ist, Versteifungselemente, insbesondere Stege, angeformt sind. Diese Ausbildung des Spritzenkopfes ermöglicht eine geringe Anhäufung von Kunststoff im Bereich des Spritzenkopfes und damit kurze Zykluszeiten beim Spritzgießen.

Vorteilhaft ist es dabei, wenn die Anlagefläche des Spritzenkopfes, an der das Kappenhemd der Schutzkappe dichtend anliegt, als Ringbund ausgebildet ist. Durch den Ringbund ist sichergestellt, daß die Schutzkappe sauber abdichtet. Die Stege der vorgeannten Ausgestaltung dienen dabei nicht nur zur Versteifung, sondern auch zur Führung des Schutzkappenrandes über den Ringbund.

Eine Weiterbildung der Erfindung ist dadurch gekennzeichnet, daß von dem Spritzenkörper zumindest der Spritzenzylinder aus einer inneren und äußeren Schicht aus verschiedenen Kunststoffen besteht. Eine derartige Ausführung mit zwei Kunststoffschichten im Bereich des Spritzenzylinders und ggf. auch im Bereich des Spritzenkopfes ist dann vorteilhaft, wenn das Präparat beispielsweise sauerstoff- oder kohlendioxidempfindlich ist. In der Regel hat ein Kunststoff, der eine gute Sperrwirkung gegen Wasserdampf aufweist, eine entsprechend schlechte Sperrwirkung gegen Gase, wie Sauerstoff und Kohlendioxid. Deshalb kann ein zweischichtiger Verbundaufbau notwendig sein.

Gemäß einer bevorzugten Ausführungsform der Erfindung besteht der Kunststoff, aus dem der Spritzenkörper hergestellt ist, aus einem zyklischen Olefin-Copolymer (COC). Die Verwendung eines zyklischen Olefin-Copolymers ist deshalb vorteilhaft, weil dieser Werkstoff eine exzellente Barriere gegen Wasserdampf bietet und gleichzeitig glasklar transparent ist.

Gemäß weiteren Ausgestaltungen der Erfindung ist es vorteilhaft durch eine weitere Schicht aus metallischem, keramischem oder glasigem Material, eine Funktionstrennung zwischen dem Kunststoff als Strukturwerkstoff, der die Form gibt und dem Beschichtungswerkstoff, der die Barriereeigenschaft bietet, vorzusehen. Bei einer Ausführungsform mit zwei Kunststoffschichten ist diese weitere Schicht zwischen den Kunststoffschichten eingebettet und deshalb gegen Verkratzen bzw. Ablösen geschützt. Eine Ausführung mit innen liegender weiterer Schicht hat den Vorteil, daß die Sperrwirkung direkt an der mit dem Präparat in Kontakt stehenden Oberfläche erzielt wird. Bei der Ausführung mit außen liegender Schicht ist gewährleistet, daß selbst im Falle des Ablösens der Schicht keine Schichtpartikel in das Präparat gelangen.

Diese verschiedenen Ausgestaltungen sowie weitere Vorteile der Erfindung werden im Zusammenhang mit den in den Zeichnungen dargestellten Ausführungsbeispielen beschrieben.

Es zeigen:
- Fig. 1: eine vollmontierte vorfüllbare Einmalspritze für medizinische Zwecke im Längsschnitt,
- Fig. 2: eine vormontierte Einheit bestehend aus Spritzenkörper, integrierter Nadel und aufgesetzter Schutzkappe im Längsschnitt,
- Fig. 3: eine weitere Ausführung des Spritzenkörpers mit umspritzter Nadel im Spritzenkopf im Längsschnitt,
- Fig. 4: eine Ausführung des Spritzenkopfes mit umspritzter, verformter Nadel im Längsschnitt,
- Fig. 5: ein Ausführung des Spritzenkopfes mit eingeklebter Nadel im Längsschnitt, und
- Fig. 6: 5 eine schematische Darstellung des Verfahrens zur Herstellung der erfindungsgemäßen Einmalspritze

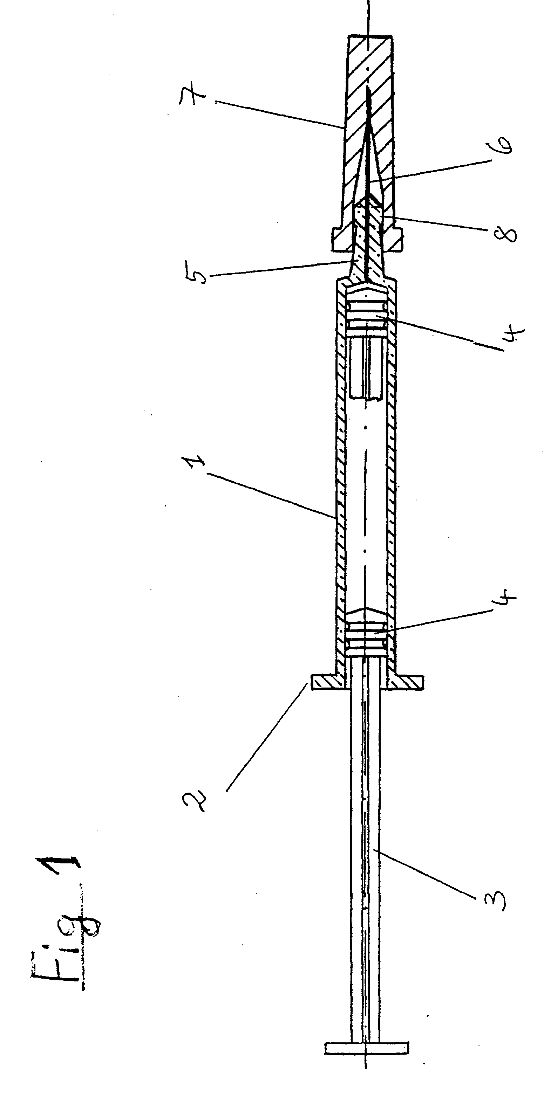

Die Figur 1 zeigt im Längsschnitt eine vorfüllbare sterile Einmalspritze für medizinische Zwecke mit einem Füllvolumen kleiner als 5 ml. Nach einer Ausgestaltung liegt das Füllvolumen der erfindungsgemäßen vorfüllbaren sterilen Einmalspritze vorzugsweise im Bereichen von 0,2 - 1 ml. Für diesen Bereich ist ein besonderer Bedarf in der Medizin vorhanden, beispielsweise zum Verabreichen von Heparin-Lösungen. Der Spritzenkörper besteht aus einem Spritzenzylinder 1, der an einem Ende eine Griffleiste 2 aufweist. In dem Spritzenzylinder 1 befindet sich ein Spritzenkolben 4, der mittels einer Kolbenstange 3 im Spritzenzylinder verschiebbar ist. Im vorgefüllten, vollmontierten Zustand der Spritze befindet sich der Spritzenkolben 4 nahe der Griffleiste 2. Nach dem Applizieren befindet sich, wie in Figur 1 ebenfalls dargestellt, der Spritzenkolben 4 am anderen Ende des Spritzenzylinders.

An diesem Ende geht der Spritzenzylinder in einen Spritzenkopf 5 über, an dem, wie noch erläutert wird, eine Injektionsnadel 6 unlösbar, d.h. nicht zerstörungsfrei lösbar, befestigt ist. Diese Injektionsnadel 6 ist durch eine Schutzkappe 7 abdeckbar. Der Spritzenkopf 5 weist dazu einen Ringbund 8 auf, wie noch deutlicher aus Fig. 3 zu entnehmen ist. An der zylindrischen Fläche des Ringbundes 8 liegt das Kappenhemd der Schutzkappe 7 dichtend an. Für die Ausbildung dieser Schutzkappe 7 stehen dem Fachmann eine Reihe von Möglichkeiten zur Verfügung. So kann beispielsweise die Schutzkappe aus einem Elastomer, vorzugsweise aus Gummi, hergestellt und die Wandstärke so groß gewählt sein, daß die Spitze der Kanüle 6 von dem Material der Schutzkappe dichtend umschlossen ist, wobei die Kanüle beim Aufsetzen der Kappe in diese einsticht. Es ist auch denkbar, eine Kunststoffkappe aus relativ hartelastischem Material vorzusehen, die innen, zumindest im Bereich der Kanülenspitze, mit einem weichelastischen dichtenden Werkstoff ausgekleidet ist. Hierdurch kann dieser weichelastische Werkstoff auf optimale Dichtungseigenschaften ausgewählt sein (Dichtfunktion), während der hartelastische Werkstoff geeigneter ist, mechanischen äußeren Beeinträchtigungen entgegenzuwirken (Schutzfunktion).

Der Spritzenzylinder 1 mit integrierter Griffleiste 2 und Spritzenkopf 5 sowie die Kolbenstange 3 bestehen aus Kunststoff, wogegen die Injektionsnadel 6, wie üblich, aus einem metallischen Werkstoff und der Spritzenkolben 4 aus einem weichelastischen Werkstoff besteht. Die Wandung des Spritzenzylinders 1 und des Spritzenkopfes 5 weisen eine Wasserdampfdurchlässigkeit von kleiner als 0,08 g/ (m² x d) bezogen auf eine Wandstärke von 500 *µ* m auf. Diese Gasdichtheit kann durch Wahl eines entsprechenden Kunststoffes erreicht werden. Je nach Anforderung ist auch ein mehrschichtiger Aufbau, wie in Fig. 3 beispielsweise dargestellt, möglich. Dieser mehrschichtige Aufbau wird später noch im einzelnen erläutert. Die Wandung der Spritze weist zumindest im Bereich des Spritzenzylinders einen glasig transparenten Abschnitt auf, so daß der Inhalt der vorgefüllten Spritze visuell auf etwaige Verunreinigungen bzw. Ausfällungen jederzeit kontrollierbar ist. Weiterhin ist das Material des Spritzenkörpers und der Schutzkappe so gewählt, daß diese gammasterilisierbar und/oder Ethylenoxid-sterilisierbar sind, ohne daß sich chemische und physikalische Eigenschaften funktionsbeeinträchtigend ändern.

Die Injektionsnadel 6 ist in dem Ausführungsbeispiel nach Fig. 1 (und Fig. 3) ohne die Verwendung eines zusätzlichen Klebstoffes allein durch die Umspritzung gehalten. Die Gestaltung der Anlagefläche des Spritzenkopfes, an der die Schutzkappe 7 dichtend anliegt, als Ringbund 8, führt zu nur geringen Materialanhäufungen im Bereich der Nadelumspritzung. Die Umspritzung der Nadel mit Kunststoff ist daher bereichsweise dünn ausgeführt, wodurch der Kunststoff nach dem Umspritzen schrumpfen kann und dadurch beim Erkalten nach dem Einspritzen des Kunststoffes in die Form eine ausreichende Dicht- und Haltewirkung erzielt wird. Dieser Effekt macht sich insbesondere bei der Ausführung nach Fig. 3 mit relativ dünner Umspritzung mit Versteifungsrippen 11 bemerkbar.

Die vorgefüllte und vollmontierte Einmalspritze nach Fig. 1 besteht aus einer vormontierten Einheit mit Spritzenkörper 1,2,5, integrierter Nadel 6 und Schutzkappe 7 einerseits sowie dem Kolben 4 mit Kolbenstange 3 andererseits. Die vormontierte Einheit ist der Deutlichkeit halber in Fig. 2 nochmals getrennt dargestellt. Sie wird im offenen Zustand mit getrenntem Kolben und Kolbenstange vom Spritzenhersteller an den Pharmazeuten geliefert, der die vormontierte Spritze befüllt und vollmontiert.

Wie in Fig. 1 dargestellt, kann die Wandung des Spritzenzylinders 1 und des Spritzenkopfes 5 homogen sein, d.h. aus einer einzigen Schicht aufgebaut sein. Der entsprechende Kunststoff muß dann sämtliche Eigenschaften aufweisen, um die vorgefüllte sterile Einmalspritze voll funktionsfähig zu halten. Er muß mit dem vorgefüllten Arzneimittel verträglich sein sowie die notwendige Festigkeit und Dampfdichtigkeit aufweisen. Der Spritzenzylinder und ggf. auch der Spritzenkopf können jedoch auch Wandungen aufweisen, die aus mehreren Schichten aus unterschiedlichen Kunststoffen gebildet sind. Im Ausführungsbeispiel der Fig. 3 besteht der Spritzenzylinder 1 aus zwei unterschiedlichen Kunststoffschichten 9 und 10. Um Einzelheiten besser hervortreten zu lassen, ist dabei in Fig. 3 ein vergrößerter Maßstab gewählt, wobei aus Darstellungsgründen der Spritzenzylinder 1 unterbrochen gezeichnet ist. Im Bereich der Nadelhalterung des Spritzenkopfes 5 sind, symmetrisch verteilt am Umfang, vier Stege 11 vorgesehen, von denen in Fig. 3 zwei ungeschnitten dargestellt sind. Die Stege setzen sich jeweils über den Ringbund 8 hinaus bis zur kegelförmigen Spitze hin fort. Diese Stege oder Rippen 11 dienen einerseits der Versteifung im Bereich der Nadelhalterung, aber auch der Führung des (nicht dargestellten) Schutzkappenrandes beim Aufsetzen.

Der in Fig. 3 dargestellte zweischichtige Aufbau des Spritzenzylinders hat den Vorteil, daß die Barriereeigenschaften der Wandung auf die Bedürfnisse des abgefüllten Präparates auf einfache Weise zugeschnitten werden können. So können beispielsweise Kunststoffe kombiniert werden, von denen der eine eine gute Barriere gegen Sauerstoffdiffusion aufweist und der andere eine gute Barriere gegen Wasserdampfdiffusion.

Als Kunststoffe mit guten Barriereeigenschaften gegen Wasserdampfdiffusion sind beispielsweise zyklische Olefin-Copolymere, Polyethylenpenten und glasklares PP, als Kunststoffe mit guten Barriereeigenschaften gegen Sauerstoffdiffusion PET, EVOH und PVDC zu nennen.

Zwischen zwei Kunststoffschichten kann auch eine Zwischenschicht aus metallischem, karamischem oder glasigem Material, wie z.B. SiOₓ, SiOₓC_{y} und TiOₓC_{y} eingebracht sein, die als Diffusionssperre wirkt. Solche Schichten haben bereits bei einer typischen Stärke von 50-300 nm eine höhere Sperrwirkung als alle bekannten Kunststoffe mit wesentlich größeren Schichtstärken. Vorgefüllte Einmalspritzen mit einer nicht organischen Zwischenschicht zwischen zwei Kunststoffschichten haben dadurch den großen Anforderungen im Hinblick auf die Barriereeigenschaften gestellt werden und sich deshalb eine größere Auswahl an in Frage kommenden Kunststoffen bietet.

Spritzenkörper aus mehreren Kunststoffschichten lassen sich durch Mehrkomponenten-Spritzgießen herstellen. Dies ist ein an sich bekanntes Verfahren, bei dem zunächst der innere Teil gespritzt wird, der dann in einem zweiten Schritt mit einem anderen Kunststoff umspritzt wird. Es ist auch denkbar, daß zwischen den beiden Schritten eine Behandlung der äußeren Oberfläche des ersten Spritzteils mit einem Plasma erfolgt, um gezielte Veränderungen der Kunststoffoberfläche mit dem Ziel der Diffusionssperre zu bewirken. Auf den ersten Körper können ebenfalls Haftvermittlerschichten zur besseren Haftung der äußeren Schicht aufgebracht werden.

Ferner können Spritzenzylinder und Spritzenkopf auch bei einschichtigem Aufbau aus Kunststoff innen mit einer Schicht aus metallischem , keramischem oder glasigem Material versehen sein. die verhindern, daß Wirkstoff sich an der Innenoberfläche der Spritze ablagert oder in den Kunststoff migriert. Diese innere Schicht kann auch so ausgebildet sein, daß sie die Gleitfähigkeit des Kolbens 4 im Inneren des Spritzenzylinders 1 erhöht. Für diesen Fall ist denkbar, daß die metallische, keramische oder glasige Schicht als Gradientenschicht aufgebaut ist, die in eine Schicht aus Fluorpolymer, wie z.B. PTFE, PFA oder FEP, übergeht, um auf die sonst übliche Innensilikonisierung des Spritzenzylinders verzichten zu können.

Die erwähnten metallischen, keramischen und glasigen Schichten sowie die Schichten aus Fluorpolymer können in einem Gasphasen-Abscheidungsverfahren (z.B. CVD, PVD, insbesondere Plasma-CVD) aufgebracht werden. Das könnte idealerweise im Spritzgießwerkzeug erfolgen. Auch sind Plasma-Polymerisationsschichten oder Veränderungen von Kunststoffen durch eine Plasmabehandlung möglich.

Die Auswahl der Schichtkombinationen muß auf die Anforderungen an das gesamte Packmittel optimiert werden. Anforderungen sind typischerweise: hoher Widerstand gegen Ein- Bzw. Ausdiffundieren von Gasen (H₂O, O₂, CO₂) sowie gegen die Eindiffusion von Kuststoffbestandteilen oder Klebstoffen des Etiketts sowie Resistenz gegen Sterilisieren durch γ-Bestrahlung, EthylenoxidBegasung oder Autoklavierung über eine Zeitdauer von 20 Minuten bei einer Temperatur von 121°C. Bei der Auswahl der Schichtabfolge ist zu berücksichtigen, daß die innere Schicht mit dem Füllgut verträglich ist (Inertheit, keine Ad- und Absorption von Bestandteilen des Füllgutes) und daß die äußere Schicht eine möglichst hohe Kratzfestigkeit aufweist.

Fig. 4 zeigt eine Ausführung des Spritzenkopfes 5 mit umspritzter Nadel 6, bei der die Nadel eine Verformung 12 aufweist. Diese Verformung schafft eine zusätzliche formschlüssige Verbindung zwischen Nadel und Halterung und sichert ein Herausziehen bzw. Hineindrücken der Nadel. Anstelle der dargestellten Verformung sind auch andere Ausgestaltungen möglich, beispielsweise eine äußere Verdickung an der Nadel.

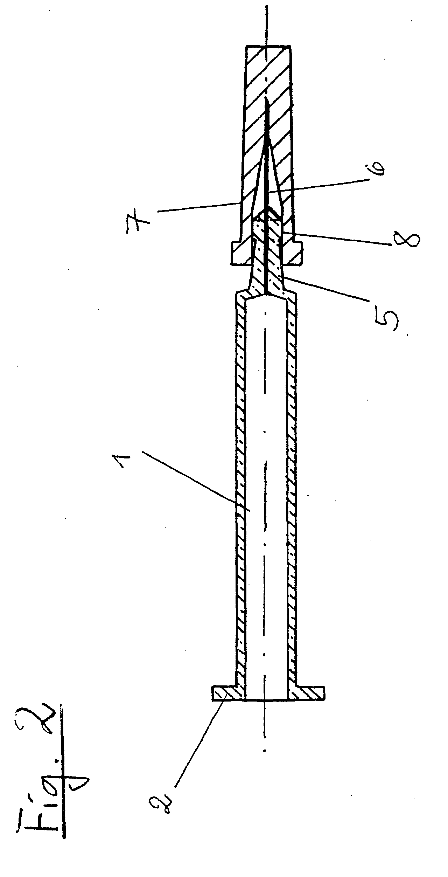

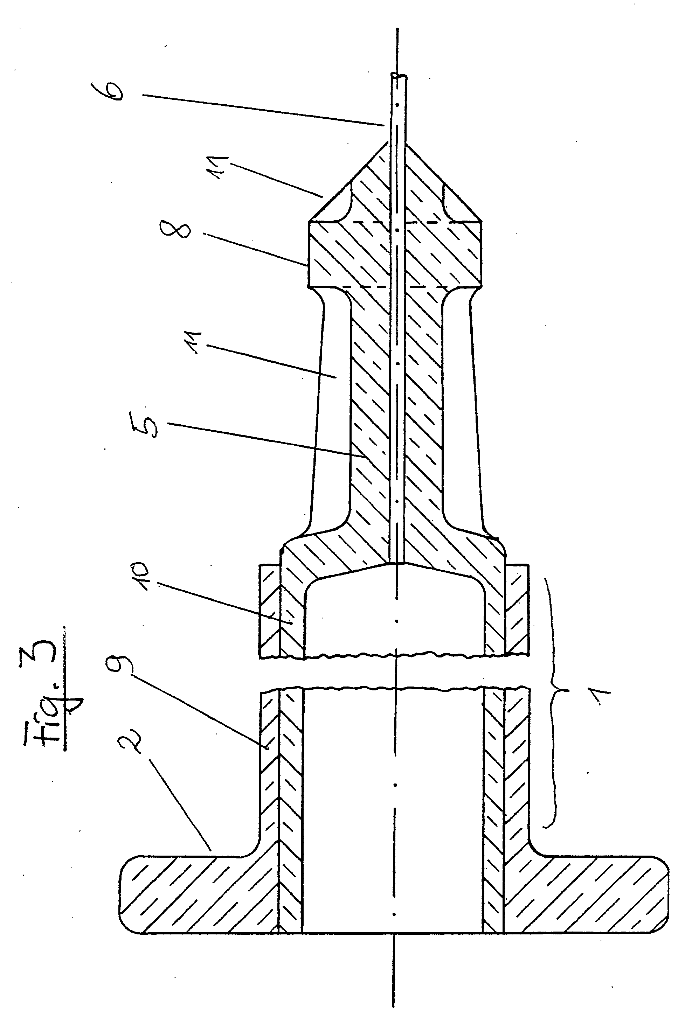

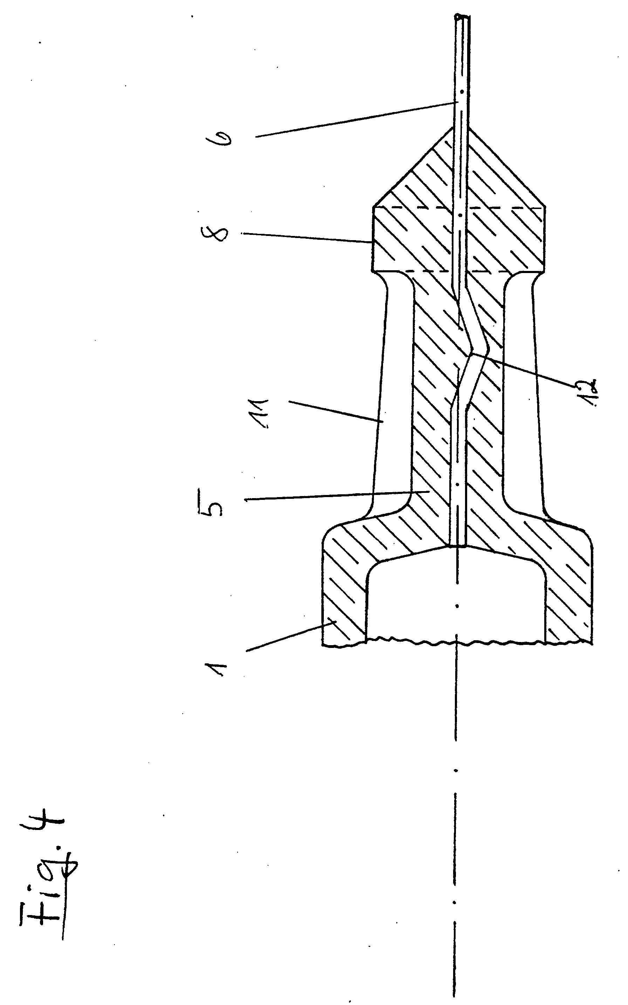

Fig. 5 zeigt eine Ausführungsform des Spritzenkopfes 5, der eine Ausnehmung 13 zur Aufnahme eines Klebstoffes, mit dem die Nadel 6 im Spritzenkopf eingeklebt ist, aufweist. Der Klebeabschnitt liegt, wie dargestellt, vorzugsweise entfernt von dem Spritzenzylinder 1 nur im vorderen Bereich des Spritzenkopfes. Dadurch wird der Kontakt zwischen Spritzeninhalt und Klebstoff weitgehend vermieden.

Die erfindungsgemäße vorfüllbare Einmalspritze ist auf relativ einfache Weise herzustellen, partikelarm zu halten und zu sterilisieren, im Vergleich zum Stand der Technik gemäß der EP 0 227 401 B1, die ein entsprechendes Verfahren zum Herstellen (und Befüllen) der großvolumigen PP-Fertigspritzen von Mallinckrodt gemäß dem eingangs bezeichneten Stand der Technik zeigt.

Im bekannten Fall erfolgt zunächst ein mechanisches Entfernen von Abfallteilschen und anderen Verunreinigungen sowie ein Sterilisieren der Verschlußkappe und des Kolbens.

Der Spritzenzylinder mit Spritzenkopf wird nach seiner Herstellung gewaschen, um Abfallteilchen und Pyrogene zu entfernen. Anschließend wird er getrocknet. Danach wird ein Gleitmittel im Innern des Spritzenzylinders aufgebracht.

Im Anschluß daran erfolgt das Aufsetzen der Schutzkappe, das befüllen des Spritzenzylinders mit der zu applisierenden Flüssigkeit, das Einsetzen des Kolbens am offenen Ende des Spritzenkörpers sowie das Sterilisieren der gesamten Einmalspritze in einem Autoklaven. Diese Verfahrensschritte laufen überwiegend beim Pharmazeuten ab, was den eigentlichen Befüllvorgang relativ aufwendig macht.

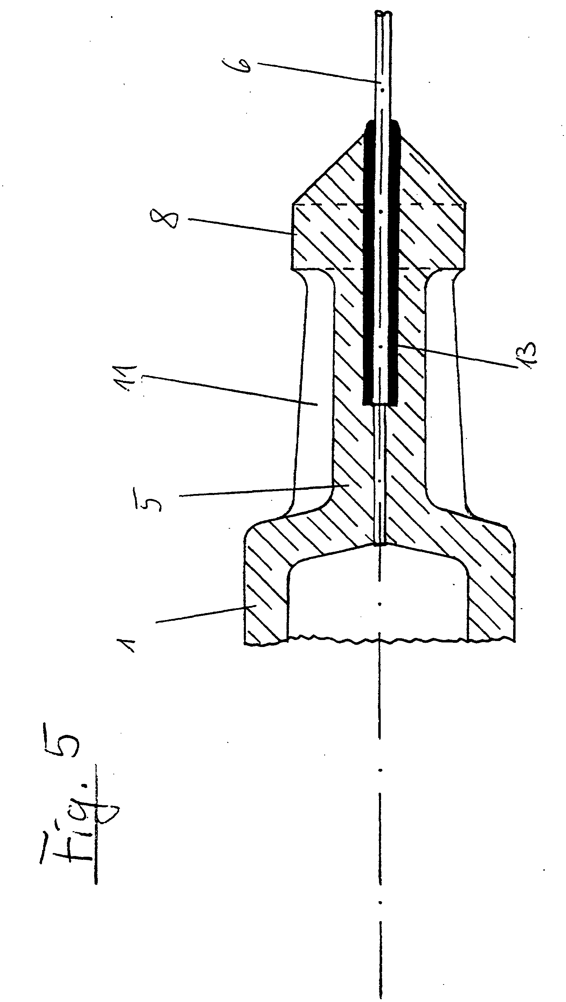

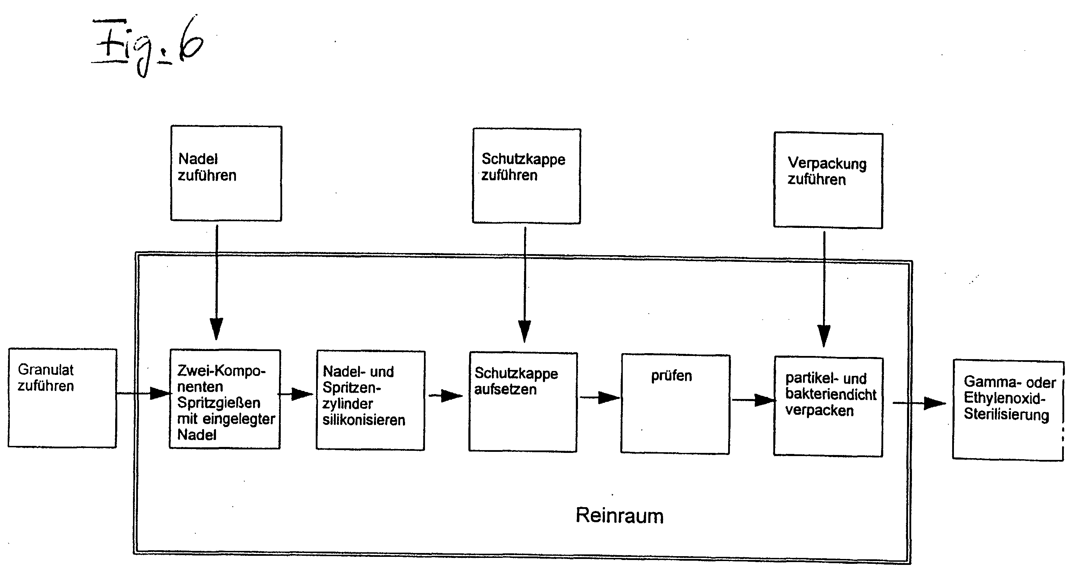

Die Herstellung der erfindungsgemäßen vorfüllbaren sterilen Einmalspritze erfolgt demgegenüber, wie in Fig. 6 dargestellt, wie folgt:
1. Herstellen des Spritzenkörpers 1 und 5 mit integrierter Injektionsnadel 6 unter Reinraumbedingungen (Partikelarmut). Dabei werden Nadeln und Schutzkappen partikelarm in den Reinraum zugeführt und am Spritzenkörper montiert. Bei der Ausführung mit umspritzter Nadel erfolgt das Spritzgießen des Spritzenkörpers mit eingelegter Nadel. Bei einem zweischichtigen Aufbau des Spritzenkörpers wird ein zwei-Komponenten Spritzgießen vorgenommen.
2. Danach erfolgt ein Aufbringen der Gleitschicht innen im Spritzenzylinder unter Reinraumbedingungen. Vorzugsweise wird Silikonöl als Gleitmittel auf den Innenkörper des Spritzenzylinders unmittelbar nach dessen Herstellung durch Sprühen, Bürsten, Tauchen oder ähnliches aufgebracht. Vorteilhaft wird dabei eine Fixierung des Gleitmittels durch Wärme (Luftkonvektion oder Strahlung) vorgesehen. Alternativ ist auch die Verwendung eines UV-polymerisierbaren Silikonöls bzw. einer entsprechenden Silikonemulsion möglich.
3. Im Anschluß daran erfolgt das Aufsetzen der Nadel-Schutzkappe 7.
4. Schließlich erfolgt ein partikel- und bakteriendichtes, magaziniertes Einsetzen der so behandelten Spritzenkörper in abgeschlossene Behälter im Reinraum und Sterilisieren durch Gammastrahlung, alternativ durch Ethylenoxidgas außerhalb des Reinraums.

Danach erfolgt - beim Pharmazeuten - das Befüllen der vormontierten Spritzeneinheit mit dem Inhaltsstoff, das Vollmontieren, d.h. das Einsetzen von Kolbenstopfen und Kolbenstange, bzw., wenn erforderlich, eine abschließende Autoklavierung und Inspektion sowie die Etikettierung und Sekundärverpackung. Vorbereitende Schritte für Füllgut, Kolbenstopfen und-stangen und weitere Packmaterialien werden prinzipiell wie beim Stand der Technik ausgeführt.

Die Vorteile des erfindungsgemäßen Verfahrens gegenüber dem bekannten Verfahren sind:
a) Das umständliche Waschen und Trocknen der Spritzenkörper entfällt, da die Kunststoffteile unter Reinraumbedingungen (Partikelarmut) hergestellt werden können. Eine Depyrogenisierung ist dabei für kleinvolumige Behältnisse, wie im Fall der Erfindung, nicht erforderlich.
b) Das Silikongleitmittel wird unmittelbar nach der Ausformung des Kunststoffes aufgebracht. Somit kann die kurz nach der Herstellung noch vorhandene erhöhte Oberlächenaktivität des Kunststoffes ausgenutzt werden, um die Haftung des Gleitmittels zu verbessern.
c) Die Sterilisierung kann auf einfache Weise noch vor dem Befüllen mit dem Inhaltsstoff durchgeführt werden. Dadurch können dem abfüllenden Pharmazeuten bereits sterile vormontierte Spritzeneinheiten zur Verfügung gestellt werden, was die Verfahrensschritte beim Pharmazeuten maßgeblich vereinfacht.

## Patentansprüche

1. Vorfüllbare partikelarme, sterile Einmalspritze für die Injektion von Präparaten mit einem Füllvolumen kleiner als 5 ml,
mit einem Spritzenkörper, der
- einen innen mit einer Gleitschicht versehenen Spritzenzylinder (1) mit einem offenen Ende zur Aufnahme eines Spritzenkolbens (4), der mittels einer Kolbenstange (3) im Spritzenzylinder bewegbar ist,
- eine Griffleiste (2)
- und der einen am Spritzenzylinder (1) angeformten, spitz zulaufenden Spritzenkopf (5) mit einer darin unmittelbar integrierten und unlösbar befestigten Injektionsnadel (6), die durch eine Schutzkappe (7) abgedeckt ist, derart, daß die Injektionsnadel (6) in die Schutzkappe (7) einsticht und damit verschlossen ist, während gleichzeitig die Innenseite der Kappe dichtend am Spritzenkopf (5) anliegt, wodurch auch die äußere Fläche der Injektionsnadel abgedichtet ist,
aufweist,
**dadurch gekennzeichnet, daß** der Spritzenkörper aus einem Kunststoff besteht, der gammasterilisierbar und/oder ethylenoxidsterilisierbar ist, ohne daß sich chemische und physikalische Eigenschaften, insbesondere Sprödigkeit und Farbe funktionsbeeinträchtigend ändern,
und bei dem die Wandungen des Spritzenzylinders (1) und des Spritzenkopfes (5) durch die Verwendung eines entsprechenden Kunststoffes in vorgegebener Wandstärke in bezug auf die Wasserdampfdichtigkeit so ausgebildet sind, daß sie eine Durchlässigkeit von kleiner als 0,08 g/(m² x d) bezogen auf eine Wandstärke von 500 *µ*m aufweisen, wobei die Wandung zumindest im Bereich des Spritzenzylinders (1) glasig transparent ausgebildet ist,
und daß die Schutzkappe gammasterilisierbar und/oder ethylenoxidsterilisierbar ist, ohne daß sich chemische und physikalische Eigenschaften funktionsbeeinträchtigend ändern.

2. Einmalspritze nach Anspruch 1, **dadurch gekennzeichnet, daß** der Bereich des Spritzenkopfes (5), in dem die Injektionsnadel (6) gehaltert ist, als Umspritzung der Nadel ausgeführt ist.

3. Einmalspritze nach Anspruch 2, **dadurch gekennzeichnet, daß** die Injektionsnadel (6) im Bereich der Umspritzung eine Profilierung oder Verformung aufweist.

4. Einmalspritze nach Anspruch 1, **dadurch gekennzeichnet, daß** die Injektionsnadel (6) in eine Ausnehmung am Spritzenkopf eingeklebt ist.

5. Einmalspritze nach einem der Ansprüche 1-4, **dadurch gekennzeichnet, daß** in dem Bereich des Spritzenkopfes (5), in dem die Injektionsnadel (6) gehalten ist, Versteifungselemente (11), insbesondere Stege, angeformt sind.

6. Einmalspritze nach einem der Ansprüche 1-5, **dadurch gekennzeichnet, daß** die Anlagefläche des Spritzenkopfes (5), an der das Kappenhemd der Schutzkappe (7) dichtend anliegt, als Ringbund (8) ausgebildet ist.

7. Einmalspritze nach einem der Ansprüche 1-6, **dadurch gekennzeichnet, daß** von dem Spritzenkörper zumindest der Spritzenzylinder (1) aus einer inneren und äußeren Schicht (9, 10) aus verschiedenen Kunststoffen besteht.

8. Einmalspritze nach einem der Ansprüche 1-7, **dadurch gekennzeichnet, daß** der Kunststoff bzw. eine der Kunststoffschichten ein zyklisches Olefin-Copolymer (COC) ist.

9. Einmalspritze nach einem der Ansprüche 7 oder 8, **dadurch gekennzeichnet, daß** zwischen den beiden Kunststoffschichten eine weitere Schicht aus metallischem, keramischem oder glasigem Material eingebracht ist.

10. Einmalspritze nach einem der Ansprüche 1-9, **dadurch gekennzeichnet, daß** der Spritzenzylinder (1) und der Spritzenkopf (5) innen mit einer Schicht aus metallischem, keramischem oder glasigem Material versehen ist.

11. Einmalspritze nach einem der Ansprüche 1-10, **dadurch gekennzeichnet, daß** der Spritzenzylinder und der Spritzenkopf außen mit einer Schicht aus metallischem, keramischem oder glasigem Material versehen ist.

12. Verfahren zum Herstellen einer vorfüllbaren partikelarmen, sterilen Einmalspritze nach Anspruch 1 oder einem der folgenden, **gekennzeichnet durch** folgende Verfahrensschritte:
a) Herstellen des Kunststoff-Spritzenkörpers **durch** Spritzgießen im Reinraum, wobei die Wandungen des Spritzenzylinders (1) und des Spritzenkopfes (5) **durch** die Verwendung eines entsprechenden Kunststoffes in vorgegebener Wandstärke in bezug auf die Wasserdampfdichtigkeit so ausgebildet werden, daß sie eine Durchlässigkeit von kleiner als 0,08 g/(m² x d) bezogen auf eine Wandstärke von 500 *µ*m aufweisen, wobei die Wandung zumindest im Bereich des Spritzenzylinders (1) glasig transparent ausgebildet ist.
b) Einbringen der Injektionsnadel und der Schutzkappe partikelarm in den Reinraum und Montage am Spritzenkörper.
c) Aufbringen der Gleitschicht innen im Spritzenzylinder im Reinraum.
d) Partikel- und bakteriendichtes Verpacken der vormontierten Spritzen-Einheit im Reinraum, magaziniert in einem Behälter, ohne daß der Spritzenkörper zuvor einem Waschvorgang unterzogen wird.
e) Sterilsieren des Behälters samt Inhalt und Schutzkappe außerhalb des Reinraums **durch** Gammabestrahlung und Ethylenoxidbegasung.

## Claims

1. Pre-fillable sterile disposable syringe with a low quantity of particles for injection of compositions, having a capacity of below 5 ml,
with a syringe body which comprises
- a syringe barrel (1) which is provided on the inside with a lubricating layer, with an open end for receiving a syringe plunger (4) which can be moved in the syringe barrel by means of a plunger rod (3),
- a grip flange (2),
- and an injection head (5) which is formed integrally on the syringe barrel (1) and tapers to a point, with an injection needle (6) integrated directly therein and secured nonreleasably, which injection needle (6) is covered by a protective cap (7) in such a way that the injection needle (6) sticks into the protective cap (7) and is thus sealed, while at the same time the inside of the cap bears sealingly on the injection head (5), as a result of which the outer surface too of the injection needle is sealed off,
**characterized in that** the syringe body is made of a plastic that can be sterilized by gamma irradiation and/or can be sterilized with ethylene oxide, without any function-impairing change in its chemical and physical properties, in particular brittleness and colour,
and in which the walls of the syringe barrel (1) and of the injection head (5) are designed, using an appropriate plastic, to have a predetermined wall thickness in relation to water vapour tightness such that they have a permeability of less than 0.08 g/(m² x d) relative to a wall thickness of 500 µm, the wall being transparent like glass at least in the area of the syringe barrel (1),
and **in that** the protective cap can be sterilized by gamma irradiation and/or can be sterilized with ethylene oxide without any function-impairing change in its chemical and physical properties.

2. Disposable syringe according to Claim 1,
**characterized in that** the area of the injection head (5) in which the injection needle (6) is mounted is designed as an injection-moulded encapsulation of the needle.

3. Disposable syringe according to Claim 2,
**characterized in that** the injection needle (6) has a profile or deformation in the area of the injection-moulded encapsulation.

4. Disposable syringe according to Claim 1,
**characterized in that** the injection needle (6) is adhesively bonded into a recess on the injection head.

5. Disposable syringe according to one of Claims 1-4,
**characterized in that** stiffening elements (11), in particular webs, are integrally formed in the area of the injection head (5) in which the injection needle (6) is held.

6. Disposable syringe according to one of Claims 1-5,
**characterized in that** the contact surface of the injection head (5) on which the skirt of the protective cap (7) bears sealingly is designed as an annular collar (8).

7. Disposable syringe according to one of Claims 1-6,
**characterized in that**, of the syringe body, at least the syringe barrel (1) is made of an inner layer and an outer layer (9, 10) of different plastics.

8. Disposable syringe according to one of Claims 1-7,
**characterized in that** the plastic, or one of the plastic layers, is a cyclic olefin copolymer (COC).

9. Disposable syringe according to either of Claims 7 and 8, **characterized in that** a further layer of metal, ceramic or glassy material is introduced between the two layers of plastic.

10. Disposable syringe according to one of Claims 1-9,
**characterized in that** the syringe barrel (1) and the injection head (5) are provided on the inside with a layer of metal, ceramic or glassy material.

11. Disposable syringe according to one of Claims 1-10, **characterized in that** the syringe barrel and the injection head are provided on the outside with a layer of metal, ceramic or glassy material.

12. Method for manufacturing a pre-fillable sterile disposable syringe with a low quantity of particles according to Claim 1 or one of the following, **characterized by** the following method steps:
a) Manufacturing the plastic syringe body by injection-moulding in a clean room, the walls of the syringe barrel (1) and of the injection head (5) being designed, using an appropriate plastic, to have a predetermined wall thickness in relation to water vapour tightness such that they have a permeability of less than 0.08 g/(m² x d) relative to a wall thickness of 500 µm, the wall being transparent like glass at least in the area of the syringe barrel (1).
b) Introducing the injection needle and the protective cap into the clean room with a low content of particles and mounting them on the syringe body.
c) Applying the lubricating layer to the inside of the syringe barrel in the clean room.
d) Packing the pre-assembled syringe unit in a particle-proof and bacteria-proof manner in the clean room, stored in a container, without the syringe body first undergoing a washing process.
e) Sterilizing the container and contents and the protective cap outside the clean room by means of gamma irradiation and ethylene oxide gas treatment.

## Revendications

1. Seringue jetable, stérile, pauvre en particules, pouvant être remplie au préalable, pour l'injection de préparations avec un volume de remplissage inférieur à 5 ml,
avec un corps de seringue présentant
- un cylindre (1) de seringue pourvu à l'intérieur d'une couche de glissement avec une extrémité ouverte pour recevoir un piston (4) de seringue qui peut être déplacé dans le cylindre de seringue au moyen d'une tige (3) de piston,
- une bordure de prise, (2)
- et une tête (5) de seringue façonnée sur le cylindre (1) de seringue, se terminant en pointe avec une aiguille d'injection (6) directement intégrée et fixée de façon inamovible dans celle-ci, laquelle aiguille est recouverte d'un capuchon (7) de protection de manière que l'aiguille d'injection (6) pénètre dans le capuchon (7) de protection et soit ainsi enfermée, pendant que simultanément l'intérieur du capuchon est ajusté de façon étanche contre la tête (5) de seringue, ce qui fait qu'également la surface extérieure de l'aiguille d'injection est rendue étanche,
**caractérisée en ce que** le corps de seringue est constitué d'une matière synthétique, qui est stérilisable par rayons gamma et/ou par de l'oxyde d'éthylène sans que des caractéristiques chimiques et physiques, en particulier la fragilité et la couleur, ne changent de façon à en compromettre la fonction et, dans lequel les parois du cylindre (1) de seringue et de la tête (5) de seringue sont conformées par l'utilisation d'une matière synthétique correspondante en une épaisseur prédéterminée, pour ce qui concerne l'étanchéité à la vapeur d'eau, de façon qu'elles présentent une perméabilité inférieure à 0,08 g/(m²x jour) rapportée à une épaisseur de paroi de 500 *µ*m, la paroi étant conformée de façon transparente comme du verre au moins dans la zone du cylindre (1) de seringue,
et **en ce que** le capuchon de protection est stérilisable par rayons gamma et/ou par de l'oxyde d'éthylène, sans que des caractéristiques chimiques et physiques ne changent de façon à en compromettre la fonction.

2. Seringue jetable selon la revendication 1,
**caractérisée en ce que** la zone de la tête (5) de seringue, dans laquelle l'aiguille d'injection (6) est maintenue, est exécutée en tant que moulage par injection autour de l'aiguille.

3. Seringue jetable selon la revendication 2,
**caractérisée en ce que** l'aiguille d'injection (6) présente dans la zone du moulage par injection autour d'elle un profilage ou une déformation.

4. Seringue jetable selon la revendication 1,
**caractérisée en ce que** l'aiguille d'injection (6) est collée à la tête de la seringue dans un évidement.

5. Seringue jetable selon une quelconque des revendications 1-4, **caractérisée en ce que** dans la zone de la tête (5) de seringue, dans laquelle l'aiguille d'injection (6) est maintenue, des éléments raidisseurs (11), en particulier des nervures, sont façonnés dessus.

6. Seringue jetable selon l'une quelconque des revendications 1-5, **caractérisée en ce que** la surface d'application de la tête (5) de seringue, contre laquelle la chemise de capuchon du capuchon (7) de protection est ajustée de façon étanche, est conformée comme bourrelet annulaire (8).

7. Seringue jetable selon une quelconque des revendications 1-6, **caractérisée en ce que**, du corps de seringue, au moins le cylindre (1) de seringue est constitué d'une couche intérieure et extérieure (9,10) en matières synthétiques différentes.

8. Seringue jetable selon une quelconque des revendications 1-7, **caractérisée en ce que** la matière synthétique ou selon le cas une des couches en matière synthétique est un copolymère oléfinique cyclique (COC).

9. Seringue jetable selon une quelconque des revendications 7 ou 8, **caractérisée en ce qu'**une autre couche en matériau métallique, céramique ou vitreux est insérée entre les deux couches en matière synthétique.

10. Seringue jetable selon l'une quelconque des revendications 1-9, **caractérisée en ce que** le cylindre (1) de seringue et la tête (5) de seringue sont pourvus à l'intérieur d'une couche en matériau métallique, céramique ou vitreux.

11. Seringue jetable selon l'une quelconque des revendications 1-10, **caractérisée en ce que** le cylindre (1) de seringue et la tête (5) de seringue sont pourvus à l'extérieur d'une couche en matériau métallique, céramique ou vitreux.

12. Procédé pour la fabrication d'une seringue jetable, stérile, pauvre en particules, pouvant être remplie au préalable, selon la revendication 1 ou selon l'une quelconque des suivantes,
**caractérisé par** les étapes suivantes de procédé:
a) fabrication du corps de seringue en matière synthétique par moulage par injection en salle blanche, les parois du cylindre (1) de seringue et de la tête (5) de seringue étant conformées par l'utilisation d'une matière synthétique correspondante en une épaisseur de paroi prédéterminée, pour ce qui concerne l'étanchéité à la vapeur d'eau, de façon qu'elles présentent une perméabilité inférieure à 0,08 g/(m² x jour) rapportée à une épaisseur de paroi de 500 *µ*m, la paroi étant conformée au moins dans la zone du cylindre (1) de seringue de façon transparente comme du verre;
b) introduction pauvre en particules de l'aiguille d'injection et du capuchon de protection dans la salle blanche et montage sur le corps de seringue;
c) application de la couche de glissement à l'intérieur du cylindre de seringue dans la salle blanche;
d) emballage étanche aux particules et aux bactéries dans la salle blanche de l'unité prémontée formant une seringue, entreposée dans un récipient sans que le corps de seringue ne soit préalablement soumis à une opération de lavage;
e) stérilisation du récipient avec son contenu et son couvercle de protection en dehors de la salle blanche par rayonnement gamma et par gazage à l'oxyde d'éthylène.
